# FASCICULE DE BREVET EUROPEEN

(11) **EP 1 010 418 B1**
(45) Date de publication et mention de la délivrance du brevet: **04.09.2002**
(21) Numéro de dépôt: 99403006.2
(22) Date de dépôt: 02.12.1999
(51) Int. Cl.: A61K 7/032, A61K 7/48

(54) **Produit de mascara comprenant un polyuréthane**
Mascara-Produkt das ein Polyurethan enthält
Mascara product comprising a polyurethane

(30) Priorité: 14.12.1998 FR 9815762
(43) Date de publication de la demande: 21.06.2000
(73) Titulaire: L'OREAL, 75008 Paris (FR)
(72) Inventeur: Piot, Bertrand, 75009 Paris (FR); de La Poterie, Valérie, 77820 Le Chatelet en Brie (FR); Auguste, Frédéric, 94550 Chevilly-Larue (FR); Bodelin, Sophie, 92170 Vanves (FR)
(74) Mandataire: Kromer, Christophe

(56) Documents cités:
- EP-A- 0 627 182
- EP-A- 0 637 600
- EP-A- 0 856 306

## Description

La présente invention a pour objet un produit de revêtement, notamment de maquillage, des fibres kératiniques, notamment des cils et des cheveux d'êtres humains, comprenant un polyuréthane. L'invention se rapporte également à un procédé de revêtement des fibres kératiniques. Le produit et le procédé de revêtement selon l'invention sont plus particulièrement destinés aux fibres kératiniques sensiblement longitudinales telles que les cils, le sourcils et les cheveux, y compris les faux-cils et les postiches. Le produit peut être un produit de maquillage, une base de maquillage, un produit à appliquer sur un maquillage, dit encore top-coat, ou bien encore un produit de traitement cosmétique des fibres kératiniques. Plus spécialement, l'invention porte sur un mascara.

Les mascaras sont couramment préparés selon deux types de formulations à base de cires : les mascaras aqueux, dits mascaras crèmes, sous forme d'émulsion de cires dans l'eau ; les mascaras anhydres ou à faible teneur en eau, dits mascaras waterproofs, sous forme de dispersions de cires dans des solvants.

Or le film de maquillage obtenu avec ces compositions appliquées sur les cils a tendance à s'effriter dans le temps : des grains se déposent et laissent des traces autour de l'oeil. De plus, le film ainsi fragilisé n'est pas résistant aux frottements, notamment des doigts et/ou à l'eau, lors de baignades ou de douches par exemple. Le maquillage n'est donc pas résistant et présente une mauvaise tenue dans le temps.

Il est connu d'employer avec les cires des polymères filmogènes qui peuvent être solubilisés ou dispersés dans 'un milieu aqueux, comme notamment décrit dans les documents FR-A-2528699 et EP-A-655234. Néanmoins, la présence de cires conduit à une mauvaise tenue du maquillage dans le temps, notamment à l'eau et aux frottements.

La présente invention a donc pour but de proposer une composition de mascara conduisant à un maquillage présentant une bonne tenue dans le temps.

La Demanderesse a constaté qu'un tel mascara pouvait être obtenu avec une composition exempte de cire et comprenant un polyuréthane filmogène. La composition est facile à appliquer et gaine bien les cils. Le maquillage est naturel, confortable et présente une bonne tenue dans le temps : le film ne s'effrite pas après une journée et se démaquille à l'eau, notamment à l'eau chaude.

De façon plus précise, l'invention a pour objet un produit de revêtement, selon la revendication 1.

L'invention a aussi pour objet un procédé re revêtement, notamment de maquillage, des fibres kératiniques, notamment des cils, caractérisée par le fait que l'on applique le long des fibres kératiniques une composition de revêtement, notamment de maquillage, du produit tel que défini précédemment.

L'invention a également pour objet l'utilisation d'une composition de revêtement, notamment de maquillage, telle que définie précédemment pour l'obtention d'un revêtement, notamment un maquillage, de bonne tenue et/ou démaquillable à l'eau.

Par "composition exempte de cire", on entend dans la présente demande une composition ne contenant pas de composé solide à température ambiante (25 °C) et ayant un point de fusion allant de 45 °C à 110 °C.

Le polyuréthane utilisé selon l'invention peut être avantageusement choisi parmi les polyester-polyuréthanes, les polyéther-polyuréthanes, les acrylique-polyuréthanes, les polyuréthanes siliconés. Le polyuréthane peut être de préférence un polyuréthane anionique.

Selon un premier mode de réalisation de l'invention, le polyuréthane peut avoir une reprise en eau inférieure ou égale à 30 %, et notamment allant de 0,5 % à 15 %. Les polyester-polyuréthanes selon l'invention présentent de telles propriétés de reprise en eau. De tels polyuréthanes permettent d'obtenir un produit de maquillage présentant une bonne tenue dans le temps, notamment supérieure à un jour, voire même deux à trois jours, et une bonne résistance à l'eau. Avantageusement, on peut utiliser des polyuréthanes, et notamment des polyester-polyuréthanes, aptes à former un film ayant une dureté allant de 40 à 200 secondes, et mieux de 50 à 170 secondes.

Selon la présente demande, on entend par "reprise en eau du polyuréthane", le pourcentage d'eau absorbé par le polyuréthane après 10 minutes d'immersion dans l'eau, à 30 °C. La reprise en eau est mesurée pour une couche de 300 µm d'épaisseur (avant séchage) déposée sur une plaque puis séchée pendant 24 heures à 30 °C et à 50 % d'humidité relative ; des morceaux d'environ 1 cm² découpés dans le film sec sont pesés (mesure de la masse M1) puis immergés dans l'eau pendant 10 minutes ; après immersion, le morceau de film est essuyé pour éliminer l'excédent d'eau en surface puis pesé (mesure de la masse M2). La différence M2 - M1 correspond à la quantité d'eau absorbée par le polymère.

La reprise en eau est égale à [ (M2 - M1) / M1 ] x 100 et est exprimée en pourcentage de poids d'eau par rapport au poids de polymère.

La dureté du film de polymère est mesurée sur un film obtenu après séchage, durant 24 heures à 30 °C et à 50 % d'humidité relative, d'une couche de 300 µm d'épaisseur (avant séchage) d'une dispersion aqueuse à 28 % de matière sèche desdites particules de polymère radicalaire. La dureté du film est mesurée selon la norme ASTM D-43-66, ou la norme NF-T 30-016 (octobre 1981), à l'aide d'un pendule de Persoz.

Selon un deuxième mode de réalisation de l'invention, le polyuréthane peut avoir une reprise en eau supérieure à 30 %, de préférence de 30 % à 150 %, et mieux de 40 % à 100 %. Les polyéther-polyuréthanes selon l'invention présentent de telles propriétés de reprise en eau. De tels polyuréthanes permettent d'obtenir un produit de maquillage bien adhérent aux cils, présentant une bonne tenue dans le temps et facile à démaquiller à l'eau, notamment à l'eau chaude à 35 - 45 °C. Avantageusement, on peut utiliser des polyuréthanes, et notamment des polyéther-polyuréthanes, aptes à former un film ayant une dureté allant de 10 à 40 secondes, et mieux de 20 à 35 secondes.

Les particules de polyuréthane dispersées dans le milieu aqueux de la composition ont généralement une taille pouvant aller de 10 nm à 200 nm.

Comme polyester-polyuréthane, on peut utiliser ceux vendus sous les dénominations "AVALURE UR-425", "AVALURE UR-430", "AVALURE UR-405", "AVALURE UR-410" par la société GOODRICH, "NEOREZ R-989" par la société ZENECA.

Comme polyéther-polyuréthane, on peut utiliser ceux vendus sous les dénominations "SANCURE 878", "AVALURE UR-450", "SANCURE 861" par la société GOODRICH.

Par acrylique-polyuréthanes, on entend des polyuréthanes comprenant au moins une chaîne de polymère acrylique, ou bien encore des polymères hybrides polyuréthane/acrylique. Comme acrylique-polyuréthanes, on peut utiliser ceux vendus sous les dénominations "UCECOAT DW 5560", "UCECOAT DW 5561","UCECOAT DW 5160","UCECOAT DW 5460","UCECOAT DW 5660" par la société UCB, "FLEXTHANE 610", "FLEXTHANE 620","FLEXTHANE 630" par la société Air Products, "SANCURE AU-4000", "SANCURE XPD-2361" par la société GOODRICH.

Le polyuréthane est présent dans la composition du produit selon l'invention en une quantité efficace pour former un film de revêtement sur les fibres kératiniques.

Le polyuréthane peut être présent dans la composition du produit selon l'invention en une teneur, en poids de matières sèches, allant de 5 % à 60 % en poids, par rapport au poids total de la composition, de préférence de 10 % à 45 % en poids, et mieux de 15 % à 35 % en poids.

Le milieu aqueux de la composition peut être constitué essentiellement d'eau. Il peut comprendre également un mélange d'eau et de solvant miscible à l'eau comme les monoalcools inférieurs ayant de 1 à 5 atomes de carbone, les cétones en C₃-C₄, les aldéhydes en C₃-C₄. Comme solvant miscible à l'eau, on peut de préférence utiliser de l'éthanol. La teneur en solvant miscible à l'eau peut aller de 0,1 % à 15 % en poids, et mieux de 1 à 8 % en poids, par rapport au poids total de la composition.

Avantageusement, la composition peut avoir une viscosité allant de 6 Pa.s (60 poises) à 15 Pa.s (150 poises), et mieux de 7 Pa.s (70 poises) à 12 Pa.s (120 poises). Une telle viscosité permet une application facile et rapide de la composition, ainsi qu'un gainage régulier sur toute la longueur des cils. La viscosité est mesurée à 25 °C avec un viscosimètre RHEOMAT RM 180 équipé d'un mobile n°4, la mesure étant effectuée après 10 minutes de rotation du mobile (temps au bout duquel on observe une stabilisation de la viscosité et de la vitesse de rotation du mobile), à un cisaillement de 200 s⁻¹.

Pour conférer à la composition du produit selon l'invention la viscosité requise pour l'application sur les fibres kératiniques, et notamment les cils, la composition peut comprendre un agent épaississant permettant d'ajuster la viscosité souhaitée.

Parmi les agents épaississants utilisables selon l'invention, on peut citer :
- les épaississants cellulosiques hydrosoluble tels que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 4400 H" par la Société Amercol,
- la gomme de guar, notamment celles vendues sous la dénomination VIDOGUM GH 175 par la société UNIPECTINE et sous la dénomination JAGUAR C par la société MEYHALL,
- la gomme de guar quaternisée vendue sous la dénomination de "Jaguar C-13-S" par la Société Meyhall,
- les gommes de guar non-ioniques comprenant des groupements hydroxyalkyle en C₁-C₆. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120 et JAGUAR HP 105 par la société MEYHALL, ou sous la dénomination GALACTASOL 40H4FD2 par la société AQUALON.
- les gommes de xanthane, de caroube, de scléroglucane, de gellane, de rhamsan, de karoya,
- les alginates, la maltodextrine, l'amidon et ses dérivés, l'acide hyaluronique et ses sels,
- les argiles, et notamment les montmorillonites, les hectorites, les laponites
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polyuréthanes associatifs.

Dans la composition selon l'invention, l'agent épaississant peut être présent une quantité efficace pour que la composition présente la viscosité telle que définie précédemment. La teneur en agent épaississant peut par exemple aller de 0,1 % à 10 % en poids, par rapport au poids total de la composition, et de préférence de 0,5 % à 5 % en poids.

La composition peut comprendre en outre d'autres ingrédients habituellement utilisés en cosmétique. De tels ingrédients peuvent être notamment choisis parmi les agents plastifiants, les agents de coalescence, les charges, les matières colorantes comme les pigments ou les colorants, les tensio-actifs, les conservateurs, les huiles, les agents cosmétiques comme les agents hydratants et les agents anti-UV qui sont bien connus de l'état de la technique. Bien entendu, l'homme du métier veillera à choisir ce ou ces éventuels additifs et/ou leur quantité, de manière telle que les propriétés avantageuses du maquillage soient conservées.

Comme charge, on peut notamment utiliser les charges habituellement employées dans les compositions de mascaras. On peut par exemple utiliser la silice pyrogénée, notamment pour l'obtention d'un maquillage plus épais sur les cils. Des charges particulièrement préférées pour la composition selon l'invention sont notamment l'amidon, comme l'amidon de riz, le talc et le polytétrafluoroéthylène : ces charges sont bien compatibles avec le milieu aqueux de la composition et sont faciles à incorporer dans la composition. De plus, elles permettent d'obtenir un film de maquillage lisse et brillant.

Les charges peuvent être présentes dans la composition en une teneur allant de 0,1 % à 6 % en poids, par rapport au poids total de la composition.

La composition peut également comprend un polymère filmogène auxiliaire, pour permettre de modifier les propriétés cosmétiques et physico-chimiques du film de maquillage. Ce polymère filmogène auxiliaire peut être sous forme dissous ou bien sous forme de particules solides dispersées dans le milieu aqueux de la composition. Comme polymère filmogène auxiliaire, on peut par exemple citer les polymères vinyliques, et notamment acryliques, ou bien encore les polyesters comme les polyesters à groupement sulfonique.

La composition de revêtement, notamment de maquillage, peut être conditionnée dans un produit d'application comprenant un réservoir et un moyen amovible pour fermer, de préférence de manière étanche, ledit réservoir.
Ledit ensemble d'application peut comprendre en outre un organe d'application de la composition de maquillage sur les fibres kératiniques, et notamment les cils, ledit organe d'application permettant le prélèvement de la composition et la restitution de la composition prélevée sur les cils. Cet organe d'application est de préférence solidaire des moyens de fermeture étanche de l'ensemble.
L'ensemble d'application peut également comprendre un organe d'essorage (ou essoreur) dudit organe d'application, l'organe d'essorage pouvant être solidaire du réservoir.

L'organe d'application peut être de préférence une brosse à mascara bien connue de l'homme du métier. Une telle brosse comprend notamment des poils disposés radialement autour d'une âme torsadée, en particulier une âme métallique. La brosse peut être de forme variée et comporter des découpes. Des brosses à mascara sont par exemple décrites dans les documents FR-A-2607373, EP-A-611170, EP-A-811336, EP-A-811337, EP-A-842620.

Dans un mode de réalisation, l'ensemble d'application peut comporter un réservoir contenant une composition de mascara telle que définie précédemment, muni d'un goulot et d'un applicateur. L'applicateur peut comporter une tige munie à une première extrémité d'un organe d'application et solidaire à une deuxième extrémité d'un élément de préhension constituant un moyen de fermeture de l'ensemble d'application.

L'ensemble d'application peut comporter, en outre, un essoreur annulaire fixé dans le goulot du réservoir et traversé par la tige, cet essoreur étant apte à essorer cette tige et/ou l'organe d'application. Avantageusement, cet essoreur a la forme d'un doigt de gant pourvu d'un orifice de passage central qui peut être éventuellement floqué. Un tel dispositif est notamment décrit dans le document FR-A-2705876.

L'invention est illustrée plus en détail dans les exemples suivants.

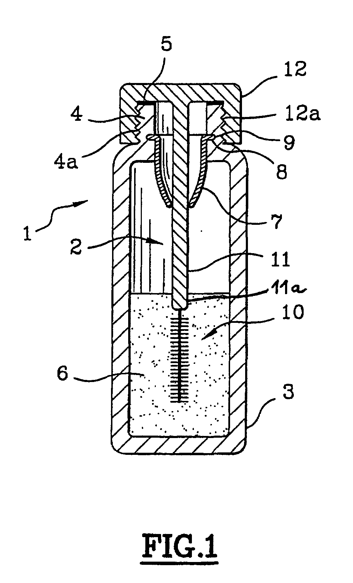

La figure 1 est une vue en élévation d'un ensemble de mascara conforme à l'invention.

En se reportant à la figure 1, on peut voir un ensemble d'application 1 de mascara comprenant un applicateur 2 et un réservoir 3, muni d'un goulot 4 fileté surmonté d'un joint d'étanchéité 5, contenant une composition de mascara 6 ayant la composition de l'exemple 1. Le réservoir 3 comporte dans son goulot 4 un essoreur 7 maintenu en place par un bourrelet 8 qui coopère avec une gorge 9 logée aussi dans le goulot 4. L'essoreur est constitué, de façon connue, en une matière souple et élastique.

L'applicateur 2 comprend un organe d'application 10 fixé à une extrémité 11a d'une tige 11. Un moyen de préhension 12 est solidaire de l'extrémité de la tige 11 opposée à l'organe d'application 10. L'organe d'application 10 est une brosse à mascara comprenant de façon connue des poils répartis radialement en nappe hélicoïdale autour d'une âme torsadée.

Le moyen de préhension 12 formant un capuchon comporte un filetage 12a qui coopère avec le filetage 4a du goulot 4 du réservoir 3. L'obturation étanche du réservoir 3 est obtenue en vissant le moyen de préhension 10 sur le goulot 4 du réservoir muni du joint 5.

Des exemples de compositions de mascara, pouvant être conditionnées dans l'ensemble d'application décrit précédemment, sont donnés ci-après.

### Exemples de composition :

### Exemple 1 :

On a préparé un mascara ayant la composition suivante :
- dispersion aqueuse de polyester-polyuréthene
   à 49 % de matières sèches
   (AVALURE UR-425 de GOODRICH) 35,8 g MA
- Hydroxyéthyl cellulose
   (Cellosize QP 4400 H d'AMERCHOL) 1,82 g
- Silice pyrogénée (AEROSIL 200 de DEGUSSA) 1,82 g
- éthanol 5 g
- propylène glycol 4,05 g
- acide citrique 0,15 g
- pigments 4 g
- conservateurs qs
- eau qsp 100 g

La mascara s'applique facilement sur les cils et conduit à un maquillage présentant une bonne tenue pendant au moins un jour, voire deux à 4 jours, résistant aux frottements des doigts et démaquillable à l'eau.

Le mascara a été testé sur un panel de 17 femmes : 12 femmes (70 %) ont constaté que le mascara a une tenue sur les cils d'au moins 3 jours après l'application.

### Exemple 2 :

On a préparé un mascara ayant la composition suivante :
- dispersion aqueuse de polyester-polyuréthane
   à 49 % de matières sèches
   (AVALURE UR-425 de GOODRICH) 24,5 g MA
- Hydroxyéthyl cellulose
   (Cellosize QP 4400 H d'AMERCHOL) 1,9 g
- Silice pyrogénée (AEROSIL 200 de DEGUSSA) 1 g
- éthanol 5 g
- propylène glycol 5 g
- pigments 5 g
- conservateurs qs
- eau qsp 100g
Le mascara s'applique facilement et adhère bien sur les cils. Le film de maquillage gaine bien les cils sur toute leur longueur et présente une bonne tenue dans le temps. De plus, le mascara se démaquille à l'eau chaude à 35 °C.

### Exemple 3 :

On a préparé un mascara ayant la composition suivante :
- dispersion aqueuse de polyéther-polyuréthane à 35 % de matières sèches
   (SANCURE 878 de GOODRICH) 24,5 g MA
- Hydroxyéthyl cellulose
   (Cellosize QP 4400 H d'AMERCHOL) 1,82 g
- Silice pyrogénée (AEROSIL 200 de DEGUSSA) 1 g
- éthanol 5 g
- propylène glycol 5 g
- pigment noir 5 g
- conservateurs qs
- eau qsp 100 g

Le polyéther-polyuréthane SANCURE 878 peut être remplacé par le polyéther-polyuréthane AVALURE UR-450 ou SANCURE 861 de GOODRICH.

Le mascara s'applique facilement sur les cils et conduit à un maquillage naturel de bonne tenue. Après deux jours, le mascara se démaquille avec de l'eau chaude à 35 °C.

### Exemple 4 :

On a préparé un mascara ayant la composition suivante :
- dispersion aqueuse de polyester-polyuréthane
   à 49 % de matières sèches
   (AVALURE UR-425 de GOODRICH) 24,5 g MA
- Hydroxyéthyl cellulose
   (Cellosize QP 4400 H d'AMERCHOL) 1,9 g
- Poudre de polytétrafluoroéthylène (CERIDUST 9205 F de HOECHST) 1 g
- éthanol 5 g
- propylène glycol 5 g
- pigments 5 g
- conservateurs qs
- eau qsp 100 g

Le mascara s'applique facilement et adhère bien sur les cils. Le film de maquillage gaine bien les cils sur toute leur longueur et présente une bonne tenue dans le temps. Le film est également lisse et brillant.

### Exemple 5 :

On a préparé un mascara ayant la composition suivante
- dispersion aqueuse de polyester-polyuréthane
   à 49 % de matières sèches
   (AVALURE UR-425 de GOODRICH) 24,5 g MA
- Hydroxyéthyl cellulose
   (Cellosize QP 4400 H d'AMERCHOL) 1,9 g
- Amidon de riz 2 g
- éthanol 5g
- propylène glycol 5 g
- pigments 5 g
- conservateurs qs
- eau qsp 100 g

Le mascara s'applique facilement et adhère bien sur les cils. Le film de maquillage présente une bonne tenue dans le temps et est lisse et brillant. De plus, le mascara se démaquille à l'eau chaude.

### Exemple 6 :

On a préparé un mascara ayant la composition suivante
- dispersion aqueuse de polyester-polyuréthane
   à 49 % de matières sèches
   (AVALURE UR-425 de GOODRICH) 24,5 g MA
- Hydroxyéthyl cellulose
   (Cellosize QP 4400 H d'AMERCHOL) 1,9 g
- Talc (LUZENAC 15 M 00 de LUZENAC) 2 g
- éthanol 5 g
- propylène glycol 5 g
- pigments 5 g
- conservateurs qs
- eau qsp 100 g

Le mascara s'applique facilement et adhère bien sur les cils. Le film de maquillage présente une bonne tenue dans le temps et est lisse et brillant.

### Exemple 7 : comparatif

On a préparé un mascara ne faisant pas partie de l'invention ayant la composition suivante :
- dispersion aqueuse de copolymère styrène/acrylate
   à 45 % de matières sèches
   (NEOCRYL A 1070 de ZENECA) 27 g MA
- Hydroxyéthyl cellulose
   (Cellosize QP 4400 H d'AMERCHOL) 1,82 g
- Silice pyrogénée (AEROSIL 200 de DEGUSSA) 1 g
- éthanol 5 g
- propylène glycol 5 g
- pigments 5g
- conservateurs qs
- eau qsp 100 g

Après l'application de la composition sur les cils, on a constaté que la tenue du film de mascara est moins bonne que celle du film de mascara obtenu avec les compositions des exemples 1 à 6.

## Revendications

1. Produit de mascara, comprenant un réservoir (3) contenant une composition de mascara, un moyen amovible de fermeture (12) destiné à fermer le réservoir et un organe d'application (10) du mascara sur les cils, **caractérisé par le fait que** la composition de mascara comprend un milieu aqueux et un polyuréthane filmogène sous forme de particules solides dispersées dans le milieu aqueux, le polyuréthane étant présent en une teneur, en poids de matières sèches, allant de 5 % à 60 % en poids, par rapport au poids total de la composition, la composition ayant une viscosité, mesurée à 25 °C, à une vitesse de cisaillement de 200 s⁻¹, allant de 5 Pa.s à 18 Pa.s, la composition étant exempte de cire.

2. Produit selon la revendication 1, **caractérisé par le fait que** le polyuréthane est choisi parmi les les polyester-polyuréthanes, les polyéther-polyuréthanes, les polyuréthanes-acrylique, les polyuréthanes siliconés.

3. Produit selon la revendication 1, **caractérisé par le fait que** le polyuréthane est choisi dans le groupe formé par les polyester-polyuréthanes et les polyéther-polyuréthanes.

4. Produit selon l'une des revendications 1 à 3, **caractérisé par le fait que** le polyuréthane est un polyuréthane anionique.

5. Produit selon l'une quelconque des revendications précédentes, **caractérisé par le fait que** le polyuréthane a une reprise en eau inférieure ou égale à 30 %.

6. Produit selon l'une quelconque des revendications 1 à 4, **caractérisé par le fait que** le polyuréthane a une reprise en eau supérieure à 30 %.

7. Produit selon l'une quelconque des revendications précédentes, **caractérisé par le fait que** le polyuréthane est présent en une teneur, en poids de matières sèches, allant de 10 % à 45 % en poids, et mieux de 15 % à 35 % en poids, par rapport au poids total de la composition.

8. Produit selon l'une quelconque des revendications précédentes, **caractérisé par le fait que** la viscosité de la composition va de 6 Pa.s à 15 Pa.s, et mieux de 7 Pa.s à 12 Pa.s.

9. Produit selon l'une quelconque des revendications précédentes, **caractérisé par le fait que** la composition comprend au moins un agent épaississant.

10. Produit selon l'une quelconque des revendications précédentes, **caractérisé par le fait que** la composition comprend au moins un agent épaississant choisi dans le groupe formé par les épaississants cellulosiques, les gommes de guar, les gommes de xanthane, de caroube, de scléroglucane, de gellane, de rhamsan, de karoya, les alginates, la maltodextrine, l'amidon et ses dérivés, l'acide hyaluronique et ses sels, les argiles, les acides polyacryliques réticulés, les poly-(métha)crylates de glycéryle, la polyvinylpyrrolidone, l'alcool polyvinylique, les polymères et les copolymères réticulés d'acrylamide, les homopolymères réticulés de chlorure de méthacryloyloxyéthyltriméthylammonium, les polymères associatifs.

11. Produit selon l'une quelconque des revendications précédentes, **caractérisé par le fait que** la composition comprend en outre au moins un ingrédient choisi dans le groupe formé par les agents plastifiants, les agents de coalescence, les charges, les matières colorantes, les tensio-actifs, les conservateurs, les huiles, les agents cosmétiques.

12. Produit selon l'une quelconque des revendications précédentes, **caractérisé par le fait que** la composition comprend en outre au moins une charge choisie dans le groupe formé par la silice pyrogénée, l'amidon, le talc et le polytétrafluoroéthylène.

13. Produit selon la revendication 11 ou 12, **caractérisé par le fait que** la charge est présente en une teneur allant de 0,1 % à 6 % en poids, par rapport au poids total de la composition.

14. Produit selon l'une quelconque des revendications précédentes, **caractérisé par le fait que** la composition comprend, en outre, un polymère filmogène auxiliaire.

15. Produit selon l'une quelconque des revendications précédentes, **caractérisé par le fait que** l'organe d'application est solidaire du moyen amovible de fermeture (12).

16. Produit selon l'une quelconque des revendications précédentes, **caractérisé par le fait que** l'organe d'application (10) est une brosse à mascara.

17. Produit selon l'une quelconque des revendications précédentes, **caractérisé par le fait qu'**il comprend en outre un organe d'essorage (7).

18. Produit selon l'une quelconque des revendications précédentes, **caractérisé par le fait qu'**il consitut un produit de maquillage, une base de maquillage, un produit à appliquer sur un maquillage, un produit de traitement cosmétique des cils.

19. Procédé de revêtement, notamment de maquillage, des cils, **caractérisé par le fait que** l'on applique le long des cils une composition de mascara selon l'une quelconque des revendications précédentes.

20. Utilisation d'un polyuréthane filmogène dans une composition de mascara, comprenant un milieu aqueux, ledit polyuréthane étant sous forme de particules solides dispersées dans ledit milieu aqueux, le polyuréthane étant présent en une teneur, en poids de matières sèches, allant de 5 % à 60 % en poids, par rapport au poids total de la composition, la composition ne contenant pas de cire et ayant une viscosité, mesurée à 25 °C, à une vitesse de cisaillement de 200 s⁻¹, allant de 5 Pa.s à 18 Pa.s, pour l'obtention d'un revêtement des cils, notamment un maquillage, de bonne tenue et/ou démaquillable à l'eau.

21. Utilisation d'un polyuréthane filmogène et d'une charge choisie dans le groupe formé par l'amidon, le talc et le polytétrafluoroéthylène, dans une composition de mascara, comprenant un milieu aqueux, ledit polyuréthane étant sous forme de particules solides dispersées dans ledit milieu aqueux, le polyuréthane étant présent en une teneur, en poids de matières sèches, allant de 5 % à 60 % en poids, par rapport au poids total de la composition, la composition ne contenant pas de cire et ayant une viscosité, mesurée à 25 °C, à une vitesse de cisaillement de 200 s⁻¹, allant de 5 Pa.s à 18 Pa.s, pour l'obtention d'un revêtement des cils, notamment un maquillage, de bonne tenue et/ou démaquillable à l'eau et/ou lisse et/ou brillant.

## Patentansprüche

1. Produkt für Mascara, das einen Behälter (3) aufweist, der eine Mascara-Zusammensetzung, ein abnehmbares Element zum Verschließen (12), das zum Verschließen des Behälters bestimmt ist, und ein Element zum Auftragen (10) der Mascara auf die Wimpern umfasst, **dadurch gekennzeichnet, dass** die Mascara-Zusammensetzung ein wässeriges Medium und ein filmbildendes Polyurethan in Form von in dem wässerigen Medium dispergierten festen Partikeln enthält, wobei das Polyurethan in einem als Trockensubstanz ausgedrückten Gewichtsanteil von. 5 bis 60 Gew.-%, bezogen auf das Gesamtgewicht der Zusammensetzung, vorliegt und wobei die Zusammensetzung bei einer Schergeschwindigkeit von 200 s⁻¹ eine bei 25 °C bestimmte Viskosität von 5 bis 18 Pa·s aufweist und wobei die Zusammensetzung frei von Wachs ist.

2. Produkt nach Anspruch 1, **dadurch gekennzeichnet, dass** das Polyurethan unter Polyester-Polyurethanen, Polyether-Polyurethanen, Acryl-Polyurethanen und siliconhaltigen Polyurethanen ausgewählt ist.

3. Produkt nach Anspruch 1, **dadurch gekennzeichnet, dass** das Polyurethan unter Polyester-Polyurethanen und Polyether-Polyurethanen ausgewählt ist.

4. Produkt nach einem der Ansprüche 1 bis 3, **dadurch gekennzeichnet, dass** das Polyurethan ein anionisches Polyurethan ist.

5. Produkt nach einem der vorhergehenden Ansprüche, **dadurch gekennzeichnet, dass** die Wasseraufnahme des Polyurethans kleiner oder gleich 30 % ist.

6. Produkt nach einem der Ansprüche 1 bis 4, **dadurch gekennzeichnet, dass** die Wasseraufnahme des Polyurethans größer 30 % ist.

7. Produkt nach einem der vorhergehenden Ansprüche, **dadurch gekennzeichnet, dass** das Polyurethan in einem als Trockensubstanz ausgedrückten Gewichtsanteil von 10 bis 15 Gew.-% und besser noch von 15 bis 35 Gew.-%, bezogen auf das Gesamtgewicht der Zusammensetzung, vorliegt.

8. Produkt nach einem der vorhergehenden Ansprüche, **dadurch gekennzeichnet, dass** die Viskosität der Zusammensetzung 6 bis 15 Pa·s und besser noch 7 bis 12 Pa·s beträgt.

9. Produkt nach einem der vorhergehenden Ansprüche, **dadurch gekennzeichnet, dass** die Zusammensetzung mindestens ein Verdickungsmittel enthält.

10. Produkt nach einem der vorhergehenden Ansprüche, **dadurch gekennzeichnet, dass** die Zusammensetzung mindestens ein Verdickungsmittel enthält, das unter cellulosehaltigen Verdickungsmitteln, Guargummen, Xanthangummi, Johannisbrotbaum-Kernmehl, Skleroglucan, Gellan, Rhamsan, Karaya-Gummi, Alginaten, Maltodextrin, Stärke, Stärkederivaten, Hyaluronsäure und ihren Salzen, Tonen, vernetzten Polyacrylsäuren, Polyglyceryl(meth)acrylaten, Polyvinylpyrrolidon, Polyvinylalkohol, vernetzten Polymeren und Copolymeren von Acrylamid, vernetzten Homopolymeren von Methacryloyloxyethyltrimethylammoniumchlorid und assoziativen Polymeren ausgewählt ist.

11. Produkt nach einem der vorhergehenden Ansprüche, **dadurch gekennzeichnet, dass** die Zusammensetzung ferner mindestens einen Zusatzstoff enthält, der unter Weichmachern, Koaleszenzmitteln, Füllstoffen, Farbmitteln, grenzflächenaktiven Stoffen, Konservierungsstoffen, Ölen und kosmetischen Wirkstoffen ausgewählt ist.

12. Produkt nach einem der vorhergehenden Ansprüche, **dadurch gekennzeichnet, dass** die Zusammensetzung ferner mindestens einen Füllstoff enthält, der unter pyrogener Kieselsäure, Stärke, Talk und Polytetrafluorethylen ausgewählt ist.

13. Produkt nach Anspruch 11 oder 12, **dadurch gekennzeichnet, dass** der Füllstoff in einem Mengenanteil von 0,1 bis 6 Gew.-%, bezogen auf das Gesamtgewicht der Zusammensetzung, vorliegt.

14. Produkt nach einem der vorhergehenden Ansprüche, **dadurch gekennzeichnet, dass** die Zusammensetzung ferner ein zusätzliches filmbildendes Polymer enthält.

15. Produkt nach einem der vorhergehenden Ansprüche, **dadurch gekennzeichnet, dass** das Element zum Auftragen fest mit dem abnehmbaren Element zum Verschließen (12) verbunden ist.

16. Produkt nach einem der vorhergehenden Ansprüche, **dadurch gekennzeichnet, dass** es sich bei dem Element zum Auftragen (10) um eine Mascarabürste handelt.

17. Produkt nach einem der vorhergehenden Ansprüche, **dadurch gekennzeichnet, dass** es ferner eine Einrichtung zum Abstreifen (7) aufweist.

18. Produkt nach einem der vorhergehenden Ansprüche, **dadurch gekennzeichnet, dass** es ein Schminkprodukt, eine Grundmasse zum Schminken, ein Produkt zum Auftragen auf eine Schminke oder ein Produkt zur kosmetischen Behandlung der Wimpern ist.

19. Verfahren zum Umhüllen und insbesondere zum Schminken der Wimpern, **dadurch gekennzeichnet, dass** eine Mascara-Zusammensetzung nach einem der vorhergehenden Ansprüche auf die gesamte Länge der Wimpern aufgetragen wird.

20. Verwendung eines filmbildenden Polyurethans in einer Mascara-Zusammensetzung, die ein wässeriges Medium enthält, wobei das Polyurethan in Form von in dem wässerigen Medium dispergierten festen Partikeln vorliegt und das Polyurethan in einem als Trockensubstanz ausgedrückten Gewichtsanteil von 5 bis 60 Gew.-%, bezogen auf das Gesamtgewicht der Zusammensetzung, enthalten ist und wobei die Zusammensetzung kein Wachs enthält und bei einer Schergeschwindigkeit von 200 s⁻¹ eine bei 25 °C bestimmte Viskosität von 5 bis 18 Pa·s aufweist, um eine Umhüllung und insbesondere eine Schminke der Wimpern zu erhalten, die gut hält und/oder mit Wasser abgenommen werden kann.

21. Verwendung eines filmbildenden Polyurethans und eines Füllstoffs, der unter Stärke, Talk und Polytetrafluorethylen ausgewählt ist, in einer Mascara-Zusammensetzung, die ein wässeriges Medium enthält, wobei das Polyurethan in Form von in dem wässerigen Medium dispergierten festen Partikeln vorliegt und das Polyurethan in einem als Trockensubstanz ausgedrückten Gewichtsanteil von 5 bis 60 Gew.-%, bezogen auf das Gesamtgewicht der Zusammensetzung, enthalten ist und wobei die Zusammensetzung kein Wachs enthält und bei einer Schergeschwindigkeit von 200 s⁻¹ eine bei 25 °C bestimmte Viskosität von 5 bis 18 Pa·s aufweist, um eine Umhüllung und insbesondere eine Schminke der Wimpern zu erhalten, die gut hält und/oder mit Wasser abgenommen werden kann und/oder glatt ist und/oder glänzt.

## Claims

1. Mascara product, comprising a reservoir (3) containing a mascara composition, a removable closure means (12) designed to close the reservoir and a member (10) for applying the mascara to the eyelashes, **characterized in that** the mascara composition comprises an aqueous medium and a film-forming polyurethane in the form of solid particles dispersed in the aqueous medium, the polyurethane being present in a content, by weight of solids, ranging from 5% to 60% by weight, relative to the total weight of the composition, the composition having a viscosity, measured at 25°C, at a shear rate of 200 s⁻¹, ranging from 5 Pa.s to 18 Pa.s, the composition being free of wax.

2. Product according to Claim 1, **characterized in that** the polyurethane is chosen from polyester-polyurethanes, polyether-polyurethanes, polyurethane-acrylics and silicone polyurethanes.

3. Product according to Claim 1, **characterized in that** the polyurethane is chosen from the group formed by polyester-polyurethanes and polyether-polyurethanes.

4. Product according to one of Claims 1 to 3, **characterized in that** the polyurethane is an anionic polyurethane.

5. Product according to any one of the preceding claims, **characterized in that** the polyurethane has a water uptake of less than or equal to 30%.

6. Product according to any one of Claims 1 to 4, **characterized in that** the polyurethane has a water uptake of greater than 30%.

7. Product according to any one of the preceding claims, **characterized in that** the polyurethane is present in a content, by weight of solids, ranging from 10% to 45% by weight, and better still from 15% to 35% by weight, relative to the total weight of the composition.

8. Product according to any one of the preceding claims, **characterized in that** the viscosity of the composition ranges from 6 Pa.s to 15 Pa.s, and better still from 7 Pa.s to 12 Pa.s.

9. Product according to any one of the preceding claims, **characterized in that** the composition comprises at least one thickener.

10. Product according to any one of the preceding claims, **characterized in that** the composition comprises at least one thickener chosen from the group formed by cellulose-based thickeners, guar gums, xanthan gum, carob gum, scleroglucan gum, gellan gum, rhamsan gum, karaya gum, alginates, maltodextrin, starch and its derivatives, hyaluronic acid and its salts, clays, crosslinked polyacrylic acids, polyglyceryl (meth)acrylates, polyvinylpyrrolidone, polyvinyl alcohol, crosslinked acrylamide polymers and copolymers, crosslinked methacryloyloxyethyltrimethyl-ammonium chloride homopolymers, and associative polymers.

11. Product according to any one of the preceding claims, **characterized in that** the composition also comprises at least one ingredient chosen from the group formed by plasticizers, coalescence agents, fillers, dyestuffs, surfactants, preserving agents, oils and cosmetic agents.

12. Product according to any one of the preceding claims, **characterized in that** the composition also comprises at least one filler chosen from the group formed by fumed silica, starch, talc and polytetrafluoroethylene.

13. Product according to Claim 11 or 12, **characterized in that** the filler is present in a content ranging from 0.1% to 6% by weight relative to the total weight of the composition.

14. Product according to any one of the preceding claims, **characterized in that** the composition also comprises an auxiliary film-forming polymer.

15. Product according to any one of the preceding claims, **characterized in that** the applicator member is integral with the removable closure means (12).

16. Product according to any one of the preceding claims, **characterized in that** the applicator member (10) is a mascara brush.

17. Product according to any one of the preceding claims, **characterized in that** it also comprises a draining member (7).

18. Product according to any one of the preceding claims, **characterized in that** it constitutes a makeup product, a makeup base, a product to be applied over a makeup, or a cosmetic treatment product for the eyelashes.

19. Process for coating and especially for making up the eyelashes, **characterized in that** a mascara composition according to any one of the preceding claims is applied along the eyelashes.

20. Use of a film-forming polyurethane in a mascara composition, comprising an aqueous medium, the said polyurethane being in the form of solid particles dispersed in the said aqueous medium, the polyurethane being present in a content, by weight of solids, ranging from 5% to 60% by weight, relative to the total weight of the composition, the composition containing no wax and having a viscosity, measured at 25°C, at a shear rate of 200 s⁻¹, ranging from 5 Pa.s to 18 Pa.s, to obtain a coat on the eyelashes, especially a coat of makeup, which has good staying power and/or which can be removed with water.

21. Use of a film-forming polyurethane and of a filler chosen from the group formed by starch, talc and polytetrafluoroethylene, in a mascara composition, comprising an aqueous medium, the said polyurethane being in the form of solid particles dispersed in the said aqueous medium, the polyurethane being present in a content, by weight of solids, ranging from 5% to 60% by weight, relative to the total weight of the composition, the composition containing no wax and having a viscosity, measured at 25°C, at a shear rate of 200 s⁻¹, ranging from 5 Pa.s to 18 Pa.s, to obtain a coat on the eyelashes, especially a coat of makeup, which has good staying power and/or which can be removed with water and/or which is smooth and/or glossy.
